(19) Europäisches Patentamt
European Patent Office
Office européen des brevets

(11) **EP 2 167 014 B1**

(12) **EUROPEAN PATENT SPECIFICATION**

(45) Date of publication and mention
of the grant of the patent:
**19.10.2011 Bulletin 2011/42**

(51) Int Cl.:
*A61K 8/00* *(2006.01)*     *C08G 77/50* *(2006.01)*
*C08L 83/12* *(2006.01)*     *C08L 83/14* *(2006.01)*

(21) Application number: **08771796.3**

(22) Date of filing: **24.06.2008**

(86) International application number:
**PCT/US2008/067989**

(87) International publication number:
**WO 2009/006091 (08.01.2009 Gazette 2009/02)**

(54) **SILICONE-ORGANIC GELS WITH POLYALKYLOXYLENE CROSSLINKED SILICONE ELASTOMERS**

SILIKON-ORGANISCHE GELE MIT POLYALKYLOXYLENVERNETZTEN SILIKONELASTOMEREN

GELS DE SILICONE ORGANIQUE AVEC ÉLASTOMÈRES DE SILICONE RÉTICULÉS AVEC DU POLYALKYLOXYLÈNE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **29.06.2007 US 937827 P**

(43) Date of publication of application:
**31.03.2010 Bulletin 2010/13**

(73) Proprietor: **Dow Corning Corporation**
**Midland, Michigan 48686-0994 (US)**

(72) Inventors:
• **KENNAN, John, Joseph**
  **Midland, MI 48640 (US)**
• **MESSNER, Kathryn, Elizabeth**
  **Midland, MI 48640 (US)**

(74) Representative: **Polypatent**
**Braunsberger Feld 29**
**51429 Bergisch Gladbach (DE)**

(56) References cited:
**US-A1- 2001 041 771     US-B1- 6 168 782**

# EP 2 167 014 B1

**Description**

<u>Technical Field</u>

This disclosure relates to silicone organic gels and gel paste compositions containing a silicone organic elastomer in a carrier fluid. The silicone organic elastomer is a reaction product of a linear or branched organohydrogensiloxane, an $\alpha, \omega$ - unsaturated polyoxyalkylene, and a hydrosilylation catalyst. The silicone organic elastomer is particularly useful to gel organic carrier fluids. The gels and gel pastes also provide enhanced compatibility with many personal and health care actives.

<u>Background</u>

Silicone elastomer gels have been used extensively to enhance the aesthetics of personal care formulations by providing a unique sensory profile upon application. Most silicone elastomer gels are obtained by a crosslinking hydrosilylation reaction of an SiH polysiloxane with another polysiloxane containing an unsaturated hydrocarbon substituent, such as a vinyl functional polysiloxane, or by crosslinking an SiH polysiloxane with a hydrocarbon diene. The silicone elastomers may be formed in the presence of a carrier fluid, such as a volatile silicone, resulting in a gelled composition. Alternatively, the silicone elastomer may be formed at higher solids content, subsequently sheared and admixed with a carrier fluid to also create gels or paste like compositions. Representative examples of such silicone elastomers are taught in U.S. Patent 5,880,210, and U.S. 5,760,116.

While silicone elastomers have provided significant advances for improving personal care formulation, they possess several shortcomings that have limited their use. For example, silicone elastomers having mostly dimethyl siloxane content are less effective for gelling organic based solvents and carrier fluids. Silicone elastomer gel compositions having high dimethyl siloxane also have limited compatibility with many personal care ingredients. For example, the widely used sunscreen agent, octyl methoxycinnamate, has limited solubility in many of these silicone elastomer gels. Another problem is the reduction of viscosity of the silicone elastomer gel in the presence of such incompatible components. Thus, there is a need to identify silicone elastomers that can gel organic solvents. Furthermore, there is a need to identify silicone elastomer gels having improved compatibilities with many personal care ingredients, while maintaining the aesthetics associated with silicone elastomer gels. To this end, there have been many attempts to improve compatibilities of silicone elastomers with various personal care ingredients wherein alkyls, polyether, amines or other organofunctional groups have been grafted onto the silicone organic elastomer backbone. Representative of such organofunctional silicone elastomers are taught in US 5,811,487 , US 5,880,210, US 6,200,581, US 5,236,986, US 6,331,604, US 6,262,170, US 6,531,540, and US 6,365,670.

US 2001/041771 A1 (KONDO HIDETOSHI [JP] ET AL) 15 November 2001 (2001-11-15) teaches a hair conditioner comprising the reaction product of a hydrogen siloxane with a di alkenyl polyether with water as carrier fluid in the form of an emulsion. US-B1-6 168 782 (LIN ZUCHEN [US] ET AL) 2 January 2001 (2001-01-02) discloses a composition in gel form for personal care products comprising the reaction product of a hydrogen siloxane with a mono alkenyl polyether in the presence of a hydrosilylation catalyst, carrier fluid and a healthcare active.

However, there is still a need to improve the compatibility of silicone elastomer based gels, and in particular, with organic based volatile fluids and personal care ingredients. Such improved compatibility should not sacrifice sensory aesthetic profiles. Furthermore, the gelling or thickening efficiency of the silicone elastomer in a carrier fluid should be maintained or improved.

The present inventors have discovered silicone organic elastomers based on certain polyoxyalkylene crosslinkers provide gelled compositions of carrier fluids efficiently. The resulting gelled compositions also possess additional benefits, such as improved compatibilities with many common personal care ingredients, while maintaining sensory aesthetics.

<u>Summary</u>

This disclosure relates to a composition comprising;

    i) a silicone organic elastomer comprising a reaction product of;

        A) an organohydrogensiloxane comprising siloxy units of average formula

$$(R^1_3SiO_{0.5})_v(R^2_2SiO)_x(R^2HSiO)_y$$

        wherein $R^1$ is hydrogen or $R^2$,

R$^2$ is a monovalent hydrocarbyl

$$v \geq 2, \; x \geq 0, \; y \geq 2,$$

B) a polyoxyalkylene comprising the average formula

$$R^3O-[(C_2H_4O)_c(C_3H_6O)_d(C_4H_8O)_c]-R^3$$

wherein n
R$^3$ is a monovalent unsaturated aliphatic hydrocarbon group containing 2 to 12 carbon atoms,
c is from 0 to 50,
d is from 0 to 100,
e is from 0 to 100,
with a proviso the ratio of (d + c)/(c + d + e) is greater than 0.5,
C) a hydrosilylation catalyst, and

ii) a carrier fluid,
wherein the composition is a gel.

The present disclosure also provides a process for making the silicone organic gels and gel pastes.
The silicone organic elastomers of the present disclosure are particularly useful to gel organic carrier fluids. The gels and gels pastes also provide enhanced compatibility with many personal and health care actives. Thus, gel or gel paste compositions containing a personal or health care active are further disclosed herein.

Detailed Description

The gel and gel paste compositions of the present disclosure contain i) a silicone organic elastomer (from the reaction of components A), B), C) and optionally D)) and ii) a carrier fluid. The gel and gel paste compositions may further contain E) a personal or health care active.

_(i) The Silicone organic elastomer_

The silicone organic elastomers of the present disclosure are obtainable as hydrosilylation reaction products of an organohydrogensiloxane, an α, ω - unsaturated polyoxyalkylene, and a hydrosilylation catalyst, components A), B), and C) respectively. The term "hydrosilylation" means the addition of an organosilicon compound containing silicon-bonded hydrogen, (such as component A) to a compound containing aliphatic unsaturation (such as component B), in the presence of a catalyst (such as component C). Hydrosilylation reactions are known in the art, and any such known methods or techniques may be used to effect the hydrosilylation reaction of components A), B), and C) to prepare the silicone organic elastomers as component i) of the present disclosure.
The silicone organic elastomer may also contain pendant, non-crosslinking moieties, independently selected from hydrocarbon groups containing 2 - 30 carbons, polyoxyalkylene groups, and mixtures thereof. Such pendant groups result from the optional addition of component D') a hydrocarbon containing 2-30 carbons having one terminal unsaturated aliphatic group, and/or component D") a polyoxyalkylene having one terminal unsaturated aliphatic group to the silicone organic elastomer via a hydrosilylation reaction.
The hydrosilylation reaction to prepare the silicone organic elastomer may be conducted in the presence of a solvent, and the solvent subsequently removed by known techniques. Alternatively, the hydrosilylation may be conducted in a solvent, where the solvent is the same as the carrier fluid described as component ii).

_A) The Organohydrogensiloxane_

Component A) of the present invention is a linear or branched organohydrogensiloxane having an average, per molecule, of at least two SiH units. As used herein, an organohydrogensiloxane is any organopolysiloxane containing a silicon-bonded hydrogen atom (SiH). Organopolysiloxanes are polymers containing siloxy units independently selected from (R$_3$SiO$_{0.5}$), (R$_2$SiO), (RSiO$_{1.5}$), or (SiO$_2$) siloxy units, where R may be any organic group. When R is a methyl group in the (R$_3$SiO$_{0.5}$), (R$_2$SiO), (RSiO$_{1.5}$), or (SiO$_2$) siloxy units of an organopolysiloxane, the siloxy units are commonly referred to as M, D, T, and Q units respectively. These siloxy units can be combined in various manners to form cyclic, linear, or branched structures. The chemical and physical properties of the resulting polymeric structures can vary. For example organopolysiloxanes can be volatile or low viscosity fluids, high viscosity fluids/gums, elastomers or rubbers, and resins.

Organohydrogensiloxanes are organopolysiloxanes having at least one SiH containing siloxy unit, that is at least one siloxy unit in the organopolysiloxane has the formula $(R_2HSiO_{0.5})$, $(RHSiO)$, or $(HSiO_{1.5})$. Thus, the organohydrogensiloxanes useful in the present invention may comprise any number of $(R_3SiO_{0.5})$, $(R_2SiO)$, $(RSiO)_{1.5}$, $(R_2HSiO_{0.5})$, $(RHSiO)$, $(HSiO_{1.5})$ or $(SiO_2)$ siloxy units, providing there are on average at least two SiH siloxy units in the molecule, and the organohydrogensiloxane is linear or branched. As used herein, "linear or branched" organohydrogensiloxane excludes cyclic organohydrogensiloxane structures. Component (A) can be a single linear or branched organohydrogensiloxane or a combination comprising two or more linear or branched organohydrogensiloxanes that differ in at least one of the following properties; structure, viscosity, average molecular weight, siloxane units, and sequence.

The organohydrogensiloxane may have the average formula;

$$(R^1_3SiO_{0.5})_v(R^2_2SiO)_x(R^2HSiO)_y$$

wherein
$R^1$ is hydrogen or $R^2$,
$R^2$ is a monovalent hydrocarbyl,
$v \geq 2$,
$x \geq 0$, alternatively $x = 1$ to 500, alternatively $x = 1$ to 200,
$y \geq 2$, alternatively $y = 2$ to 200, alternatively $y = 2$ to 100.

$R^2$ may be a substituted or unsubstituted aliphatic or aromatic hydrocarbyl. Monovalent unsubstituted aliphatic hydrocarbyls are exemplified by, but not limited to alkyl groups such as methyl, ethyl, propyl, pentyl, octyl, undecyl, and octadecyl and cycloalkyl groups such as cyclohexyl. Monovalent substituted aliphatic hydrocarbyls are exemplified by, but not limited to halogenated alkyl groups such as chloromethyl, 3-chloropropyl, and 3,3,3-trifluoropropyl. The aromatic hydrocarbon group is exemplified by, but not limited to, phenyl, tolyl, xylyl, benzyl, styryl, and 2-phenylethyl.

In one embodiment, the organohydrogensiloxane may contain additional siloxy units and have the average formula

$$(R^1_3SiO_{0.5})_v(R^2_2SiO)_x(R^2HSiO)_y(R^2SiO_{1.5})_z,$$

$$(R^1_3SiO_{0.5})_v(R^2_2SiO)_x(R^2HSiO)_y(SiO_2)_w,$$

$$(R^1_3SiO_{0.5})_v(R^2_2SiO)_x(R^2HSiO)_y(SiO_2)_w(R^2SiO_{1.5})_z$$

or any mixture thereof,
where
$R^1$ is hydrogen or $R^2$,
$R^2$ is a monovalent hydrocarbyl,
and $v \geq 2$, $w \geq 0$, $x \geq 0$, $y \geq 2$, and z is $\geq 0$.

In one embodiment, the organohydrogensiloxane is selected from a dimethyl, methylhydrogen polysiloxane having the average formula;

$$(CH_3)_3SiO[(CH_3)_2SiO]_x[(CH_3)HSiO]_ySi(CH_3)_3$$

where $x \geq 0$, alternatively, $x = 1$ to 500, alternatively $x = 1$ to 200,
and $y \geq 2$, alternatively, $y = 2$ to 200, alternatively $y = 2$ to 100.

In one embodiment, the organohydrogensiloxane is mixture of dimethyl, methylhydrogen polysiloxane having the average formula $(CH_3)_3SiO[(CH_3)_2SiO]_x[(CH_3)HSiO]_ySi(CH_3)_3$ and SiH terminal dimethyl polysiloxane having the average formula $H(CH_3)_2SiO[(CH_3)_2SiO]_xSi(CH_3)_2H$ where x and y are as defined above. The amount of each organohydrogensiloxane in the mixture may vary, or alternatively may be such that in the mixture 0 to 85 wt %, alternatively 10 to 70 wt %, alternatively 20 to 60 wt % or alternatively 30 to 50 wt % of the total SiH in the mixture is from the SiH content of the SiH terminal dimethyl polysiloxane.

Methods for preparing organohydrogensiloxanes are well known, and many are sold commercially.

*B) The Polyoxyalkylene*

Component B) is a polyoxyalkylene having an average formula

$$R^3O-[(C_2H_4O)_c(C_3H_6O)_d(C_4H_8O)_c]-R^3$$

wherein
$R^3$ is a monovalent unsaturated aliphatic hydrocarbon group containing 2 to 12 carbon atoms,
c is from 0 to 50, alternatively 0 to 10, or alternatively less than 2,
d is from 0 to 100, alternatively 1 to 100, or alternatively 5 to 50,
e is from 0 to 100, alternatively 0 to 50, or alternatively 0 to 30,
with a proviso the ratio of (d + e)/(c + d + e) is greater than 0.5. alternatively greater than 0.8.
or alternatively greater than 0.95.

The polyoxyalkylene useful as component B) can be any polyoxyalkylene that is terminated at each molecular chain end (i.e. alpha and omega positions) with a unsaturated aliphatic hydrocarbon group containing 2 to 12 carbon atoms. The polyoxyalkylene may result from the polymerization of ethylene oxide, propylene oxide, butylene oxide, 1,2-epoxyhexane, 1, 2-epoxyoctane, cyclic epoxides such as cyclohexene oxide or exo-2,3-epoxynorborane. The polyoxyalkylene group may comprise oxyethylene units ($C_2H_4O$), oxypropylene units ($C_3H_6O$), oxybutylene units ($C_4H_8O$), or mixtures thereof. Typically, the polyoxyalkylene group comprises a majority of oxypropylene or oxybutylene units, as defined on a molar basis and indicated in the above formula by the c, d, and e subscripts. The unsaturated aliphatic hydrocarbon group can be an alkenyl or alkynyl group. Representative, non- limiting examples of the alkenyl groups are shown by the following structures; $H_2C=CH-$, $H_2C=CHCH_2-$, $H_2C=C(CH_3)CH_2-$, $H_2C=CHCH_2CH_2-$, $H_2C=CHCH_2CH_2CH_2-$, and $H_2C=CHCH_2CH_2CH_2CH_2-$. Representative, non- limiting examples of alkynyl groups are shown by the following structures; $HC\equiv C-$, $HC\equiv CCH_2-$. $HC\equiv CC(CH_3)-$, $HC\equiv CC(CH_3)_2-$, $HC\equiv CC(CH_3)_2CH_2-$.

In one embodiment, the polyoxyalkylene is selected from

$$H_2C=CHCH_2O[C_3H_6O]_dCH_2CH=CH_2,$$

$$H_2C=C(CH_3)CH_2O[C_3H_6O]_dCH_2C(CH_3)=CH_2$$

$$HC\equiv CCH_2O[C_3H_6O]_dCH_2C\equiv CH, \text{ or}$$

$$HC\equiv CC(CH_3)_2O[C_3H_6O]_dC(CH_3)_2C\equiv CH$$

where d is as defined above.

Polyoxyalkylenes having an unsaturated aliphatic hydrocarbon group at each molecular terminal are known in the art, and many are commercially available. Polyoxyalkylenes having an unsaturated aliphatic hydrocarbon group at each molecular terminal are commercially available from NOF (Nippon Oil and Fat, Tokyo, Japan) and Clariant Corp. (Charlottesville, NC).

The amounts of components A) and B) used in the hydrosilylation reaction may vary. Typically, the molar ratio of the SiH units of component A) to the aliphatic unsaturated groups of component B) ranges from 10/1 to 1/10, alternatively from 5/1 to 1/5, or alternatively from 2/1 to 1/2. In one embodiment, the molar ratio of the unsaturated aliphatic hydrocarbon groups in B) to the SiH units in A) is greater than 1.

*(C) The Hydrosilylation Catalyst*

Component (C) comprises any catalyst typically employed for hydrosilylation reactions. It is preferred to use platinum group metal-containing catalysts. By platinum group it is meant ruthenium, rhodium, palladium, osmium, iridium and platinum and complexes thereof. Platinum group metal-containing catalysts useful in preparing the compositions of the present invention are the platinum complexes prepared as described by Willing, U. S. Pat. No. 3,419,593, and Brown et al, U. S. Pat. No. 5,175,325, each of which is hereby incorporated by reference to show such complexes and their preparation. Other examples of useful platinum group metal-containing catalysts can be found in Lee et al., U. S. Pat. No. 3,989,668; Chang et al., U. S. Pat. No. 5,036,117; Ashby, U. S. Pat. No. 3,159,601; Lamoreaux, U. S. Pat. No. 3,220,972; Chalk et al., U. S. Pat. No. 3,296,291; Modic, U. S. Pat. No. 3,516,946; Karstedt, U. S. Pat. No. 3,814,730; and Chandra et al., U. S. Pat. No. 3,928,629 all of which are hereby incorporated by reference to show useful platinum group metal-containing catalysts and methods for their preparation. The platinum group-containing catalyst can be platinum group metal, platinum group metal deposited on a carrier such as silica gel or powdered charcoal, or a compound or complex of a platinum group metal. Preferred platinum-containing catalysts include chloroplatinic acid, either in hexahydrate form or anhydrous form, and or a platinum-containing catalyst which is obtained by a method comprising reacting chloroplatinic acid with an aliphatically unsaturated organosilicon compound such as divinyltetramethyldisiloxane, or alkene-platinum-silyl complexes as described in U.S. Patent Application No. 10/017229, filed December 7, 2001, such as $(COD)Pt(SiMeCl_2)_2$, where COD is 1,5-cyclooctadiene and Me is methyl. These alkene-platinum-silyl complexes may be prepared, for example by mixing 0.015 mole $(COD)PtCl_2$ with 0.045 mole COD and 0.0612 moles $HMeSiCl_2$.

The appropriate amount of the catalyst will depend upon the particular catalyst used. The platinum catalyst should be present in an amount sufficient to provide at least 2 parts per million (ppm), alternatively 4 to 200 ppm of platinum based on total weight percent solids (all non-solvent ingredients) in the composition. Typically, the platinum is present in an amount sufficient to provide 4 to 150 weight ppm of platinum on the same basis. The catalyst may be added as a single species or as a mixture of two or more different species.

_D) Optional components containing one terminal unsaturated aliphatic hydrocarbon group_

The silicone organic elastomer may also contain pendant, non-crosslinking moieties, independently selected from hydrocarbon groups containing 2 - 30 carbons, polyoxyalkylene groups, and mixtures thereof. These groups are formed on the silicone organic elastomer via a hydrosilylation reaction by the addition of component D) an organic compound having one terminal unsaturated aliphatic hydrocarbon group. Component D) may be selected from D') a hydrocarbon containing 6-30 carbons having one terminal unsaturated aliphatic hydrocarbon group, and/or component D") a polyoxyalkylene having one terminal unsaturated aliphatic group.

The addition of component D) can alter the resulting chemical and physical properties of the silicone organic elastomer. For example, selecting D' will result in the addition of hydrocarbon groups to the silicone organic elastomer, thus adding more hydrophobic character to the silicone organic elastomer. Conversely, selecting a polyoxyalkylene having a majority of ethylene oxide units will result in a silicone organic elastomer having increased hydrophilicity, which can subsequently incorporate water or hydrophilic components with the silicone organic elastomer to form dispersions or pastes.

The unsaturated aliphatic hydrocarbon group in D' or D" can be an alkenyl or alkynyl group. Representative, non- limiting examples of the alkenyl groups are shown by the following structures; $H_2C=CH-$, $H_2C=CHCH_2-$, $H_2C=C(CH_3)CH_2-$, $H_2C=CHCH_2CH_2-$, $H_2C=CHCH_2CH_2CH_2-$, and $H_2C=CHCH_2CH_2CH_2CH_2-$. Representative, non- limiting examples of alkynyl groups are shown by the following structures; $HC\equiv C-$, $HC\equiv CCH_2-$, $HC\equiv CC(CH_3)-$, $HC\equiv CC(CH_3)_2-$, $HC\equiv CC(CH_3)_2CH_2-$.

Component D'), the hydrocarbon containing 6-30 carbons having one terminal unsaturated aliphatic group, may be selected from alpha olefins such as 1-hexene, 1-octene, 1-decene, 1- undecene, 1-decadecene, and similar homologs. Component D') may also be selected from aryl containing hydrocarbons such as alpha methyl styrene.

Component D") may be selected from those polyoxyalkylenes having the average formula

$$R^3O-[(C_2H_4o)_{c'}(C_3H_6O)_{d'}(C_4H_8O)_c]-R^4$$

where $R^3$ is a monovalent unsaturated aliphatic hydrocarbon group containing 2 to 12 carbon atoms,
c' is from 0 to 100, d' is from 0 to 100. e is from 0 to 100,
providing the sum of c', d', und e is > 0.
$R^4$ is hydrogen, an acyl group, or a monovalent hydrocarbon group containing 1 to 8 carbons. Representative, non-limiting examples of polyoxyalkylenes, useful as component D") include;

$$H_2C=CHCH_2O(C_2H_4O)_{c'}H$$

$$H_2C=CHCH_2O(C_2H_4O)_{c'}CH_3$$

$$H_2C=CHCH_2O(C_2H_4O)_{c'}C(O)CH_3$$

$$H_2C=CHCH_2O(C_2H_4O)_{c'}(C_3H_6O)_{d'}H$$

$$H_2C=CHCH_2O(C_2H_4O)_{c'}(C_3H_6O)_{d'}CH_3$$

$$H_2C=CHCH_2O(C_2H_4O)_{c'}C(O)CH_3$$

$$H_2C=C(CH_3)CH_2O(C_2H_4O)_{c'}H$$

$$H_2C=CC(CH_3)_2O(C_2H_4O)_{c'}H$$

$$H_2C=C(CH_3)CH_2O(C_2H_4O)_{c'}CH_3$$

$$H_2C=C(CH_3)CH_2O(C_2H_4O)_{c'}C(O)CH_3$$

$$H_2C=C(CH_3)CH_2O(C_2H_4O)_{c'}(C_3H_6O)_{d'}H$$

$$H_2C=C(CH_3)CH_2O(C_2H_4O)_{c'}(C_3H_6O)_{d'}CH_3$$

$$H_2C=C(CH_3)CH_2O(C_2H_4O)_{c'}C(O)CH_3$$

$$HC\equiv CCH_2O(C_2H_4O)_{c'}H$$

$$HC\equiv CCH_2O(C_2H_4O)_{c'}CH_3$$

$$HC\equiv CCH_2O(C_2H_4O)_{c'}C(O)CH_3$$

$$HC\equiv CCH_2O(C_2H_4O)_{c'}(C_3H_6O)_{d'}H$$

$$HC\equiv CCH_2O(C_2H_4O)_{c'}(C_3H_6O)_{d'}CH_3$$

$$HC\equiv CCH_2O(C_2H_4O)_{c'}C(O)CH_3$$

where c' and d' are as defined above.

The polyether may also be selected from those as described in US 6,987,157, which is herein incorporated by reference for its teaching of polyethers.

Components D' or D'' may be added to the silicone organic elastomer either during formation (i.e. simultaneously reacting components A), B), C) and D), in a first reaction (for example reacting a partial quantity of SiH groups of component A) with C) and D), followed by further reaction with B) or subsequently added to a formed silicone organic elastomer having SiH content (for example, from unreacted SiH units present on the silicone organic elastomer).

The amount of component D' or D'' used in the hydrosilylation reaction may vary, providing the molar quantity of the total aliphatic unsaturated groups present in the reaction from components B) and D) is such that the molar ratio of the SiH units of component A) to the aliphatic unsaturated groups of components B) and D) ranges from 10/1 to 1/10.

*(ii) The Carrier Fluid*

The silicone organic elastomers (i) are contained in a carrier fluid (ii) to provide the present silicone-organic gel compositions. Typically, the carrier fluid is the solvent used for conducting the hydrosilylation reaction to form the silicone organic elastomer. Suitable carrier fluids include, organic liquids (oils and solvents), silicones and mixtures of these.

Typically, the carrier fluid is an organic liquid. Organic liquids includes those considered oils or solvents. The organic liquids are exemplified by, but not limited to, aromatic hydrocarbons, aliphatic hydrocarbons, alcohols, aldehydes, ketones, amines, esters, ethers, glycols, glycol ethers, alkyl halides and aromatic halides. Hydrocarbons include, isododecane, isohexadecane, Isopar L (C11-C13), IsoparH (C11-C12), hydrogentated polydecene. Ethers and esters include, isodecyl neopentanoate, neopentylglycol heptanoate, glycol distearate, dicaprylyl carbonate, diethylhexyl carbonate, propylene glycol n butyl ether, ethyl-3 ethoxypropionate, propylene glycol methyl ether acetate, tridecyl neopentanoate, propylene glycol methylether acetate (PGMEA), propylene glycol methylether (PGME). octyldodecyl neopentanoate, diisobutyl adipate, diisopropyl adipate, propylene glycol dicaprylate / dicaprate, and octyl palmitate. Additional organic carrier fluids suitable as a stand alone compound or as an ingredient to the carrier fluid include fats, oils, fatty acids, and fatty alcohols.

The carrier fluid may also be a low viscosity organopolysiloxane or a volatile methyl siloxane or a volatile ethyl siloxane or a volatile methyl ethyl siloxane having a viscosity at 25°C in the range of 1 to 1,000 mm$^2$/sec such as hexamethylcyclotrisiloxane, octamethylcyclotetrasiloxane, decamethylcyclopentasiloxane, dodecamethylcyclohexasiloxane, octamethyltrisiloxane, decamethyltetrasiloxane, dodecamethylpentasiloxane, tetradecamethylhexasiloxane, hexadeamethylheptasiloxane, heptamethyl-3-{((trimethylsilyl)oxyl)}trisiloxane, hexamethyl-3,3,bisl{(trimethylsilyl)oxy}trisiloxane pentamethyl{(trimethylsilyl)oxy}cyclotrisiloxane as well as polydimethylsiloxanes, polyethylsiloxanes, polymethylethylsiloxanes, polymethylphenylsiloxanes, polydiphenylsiloxanes, and any mixtures thereof.

The amount of i) silicone organic elastomer and ii) carrier fluid is such that the composition contains

2 - 95 weight percent,
alternatively 5 to 95 weight percent
alternatively 10 to 90 weight percent of i) the silicone organic elastomer, and
5 - 98 weight percent,
alternatively 95 to 5 weight percent
alternatively 90 to 10 weight percent of ii) the carrier fluid. providing the sum of components i) and ii), and any other ingredients or components present in the composition, sum to 100 weight percent.

*Process for preparing the gel composition*

The gel compositions may be prepared by the processes of the present disclosure. The disclosed process involves;

I) reacting;

A) an organohydrogensiloxane comprising siloxy units of average formula

$$(R^1_3SiO_{0.5})_v(R^2_2SiO)_x(R^2HSiO)_y$$

wherein $R^1$ is hydrogen or $R^2$,
$R^2$ is a monovalent hydrocarbyl
$v \geq 2$, $x \geq 0$, $y \geq 2$,
B) a polyoxyalkylene comprising the average formula

$$R^3O-[(C_2H_4O)_c(C_3H_6O)_d(C_4H_8O)_e]-R^3$$

wherein
$R^3$ is a monovalent unsaturated aliphatic hydrocarbon group containing 2 to 12 carbon atoms,
c is from 0 to 50,
d is from 0 to 100,
e is from 0 to 100,
with a proviso the ratio of $(d + e)/(c + d + e)$ is greater than 0.5,
C) a hydrosilylation catalyst,
and optionally
D') a hydrocarbon containing 6-30 carbons having one terminal unsaturated aliphatic hydrocarbon group.
D") a polyoxyalkylene having one terminal unsaturated aliphatic group, or mixtures of D') and D"),
in the presence of

ii) a carrier fluid,
to form a gel.

Components A), B), C), and D) and the carrier fluid ii), and the quantities used in the process are the same as described above. The order of addition of components A), B), C), and optionally D) in step I) is not critical. Typically, components A), B), and optionally D) are combined with the carrier fluid with mixing, and the mixture heated to 70-90°C. Then, the catalyst C) is added to cause the hydrosilylation reaction. Alternatively, components A) and D) are combined, mixed, and heated to 70-90°C, catalyst C) added, and subsequently component B) is added.

The process of the present disclosure may further include the step of mixing an organovinylsiloxane to the gel composition. Organovinylsiloxanes are organopolysiloxanes having at least one vinyl (Vi is $CH_2$=CH-) containing siloxy unit, that is at least one siloxy unit in the organopolysiloxane has the formula $(R_2ViSiO_{0.5})$, $(RViSiO)$, or $(ViSiO_{1.5})$. The addition of an organovinylsiloxane may enhance the long term stability of the gel composition. Although not wishing to be bound by any theory, the present inventors believe the addition of the organovinylsiloxane may react with residual SiH that may remain on the silicone organic elastomer.

*E) The Personal or Health Care Active*

Optional component E) is an active selected from any personal or health care active. As used herein, a "personal care active" means any compound or mixtures of compounds that are known in the art as additives in the personal care formulations that are typically added for the purpose of treating hair or skin to provide a cosmetic and/or aesthetic benefit. A "healthcare active" means any compound or mixtures of compounds that are known in the art to provide a pharmaceutical or medical benefit. Thus, "healthcare active" include materials consider as an *active ingredients* or *active drug ingredient* as generally used and defined by the United States Department of Health & Human Services Food and Drug Administration, contained in Title 21, Chapter 1, of the Code of Federal Regulations, Parts 200-299 and Parts 300-499.

In one embodiment, component E) is a sunscreen agent. The sunscreen agent can be selected from any sunscreen agent known in the art to protect skin from the harmful effects of exposure to sunlight. The sunscreen compound is typically chosen from an organic compound, an inorganic compound, or mixtures thereof that absorbs ultraviolet (UV) light. Thus, representative non limiting examples that can be used as the sunscreen agent include; Aminobenzoic Acid, Cinoxate, Diethanolamine Methoxycinnamate, Digalloyl Trioleate, Dioxybenzone, Ethyl 4-[bis(Hydroxypropyl)] Aminobenzoate, Glyceryl Aminobenzoate, Homosalate, Lawsone with Dihydroxyacetone, Menthyl Anthranilate, Octocrylene, Octyl Methoxycinnamate, Octyl Salicylate, Oxybenzone, Padimate O, Phenylbenzimidazole Sulfonic Acid, Red Petrolatum, Sulisobenzone, Titanium Dioxide, and Trolamine Salicylate, cetaminosalol, Allatoin PABA, Benzalphthalide, Benzophenone, Benzophenone 1-12, 3-Benzylidene Camphor, Benzylidenecamphor Hydrolyzed Collagen Sulfonamide, Benzylidene Camphor Sulfonic Acid, Benzyl Salicylate, Bornelone, Bumetriozole, Butyl Methoxydibenzoylmethane, Butyl PABA, Ceria/Silica, Ceria/Silica Talc, Cinoxate, DEA-Methoxycinnamate, Dibenzoxazol Naphthalene, Di-t-Butyl Hydroxybenzylidene Camphor, Digalloyl Trioleate, Diisopropyl Methyl Cinnamate, Dimethyl PABA Ethyl Cetearyldimonium Tosylate, Dioctyl Butamido Triazone, Diphenyl Carbomethoxy Acetoxy Naphthopyran, Disodium Bisethylphenyl Tiamminotriazine Stilbenedisulfonate, Disodium Distyrylbiphenyl Triaminotriazine Stilbenedisulfonate, Disodium Distyrylbiphenyl Disulfonate, Drometrizole, Drometrizole Trisiloxane, Ethyl Dihydroxypropyl PABA, Ethyl Diisopropylcinnamate, Ethyl Methoxycinnamate, Ethyl PABA, Ethyl Urocanate, Etrocrylene Ferulic Acid, Glyceryl Octanoate Dimethoxycinnamate, Glyceryl PABA, Glycol Salicylate, Homosalate, Isoamyl p-Methoxycinnamate, Isopropylbenzyl Salicylate, Isopropyl Dibenzolylmethane, Isopropyl Methoxycinnamate, Menthyl Anthranilate, Menthyl Salicylate, 4-Methylbenzylidene, Camphor, Octocrylene, Octrizole, Octyl Dimethyl PABA. Octyl Methoxycinnamate, Octyl Salicylate, Octyl Triazone, PABA, PEG-25 PABA, Pentyl Dimethyl PABA, Phenylbenzimidazole Sulfonic Acid, Polyacrylamidomethyl Benzylidene Camphor, Potassium Methoxycinnamate, Potassium Phenylbenzimidazole Sulfonate, Red Petrolatum, Sodium Phenylbenzimidazole Sulfonate, Sodium Urocanate, TEA-Phenylbenzimidazole Sulfonate, TEA-Salicylate, Terephthalylidene Dicamphor Sulfonic Acid, Titanium Dioxide, Zinc Dioxide, Serium Dioxide, TriPABA Panthenol, Urocanic Acid, and VA/Crotonates/Methacrytoxybenzophenone-1 Copolymer.

The sunscreen agent can be a single one or combination of more than one. Alternatively, the sunscreen agent is a cinnamate based organic compound, or alternatively, the sunscreen agent is octyl methoxycinnamate, such as Uvinul® MC 80 an ester of paramethoxycinnamic acid and 2-ethylhexanol.

The amount of component E) present in the silicone gel composition may vary, but typically range as follows;

.05 to 50 wt%, alternatively 1 to 25 wt %, or alternatively 1 to 10 wt%, based on the total amount by weight of silicone organic elastomer gel composition.

The active, component E), may be added to the silicone gel composition either during the making of the silicone organic elastomer (pre-load method), or added after the formation of the silicone organic elastomer gel (post load method).

*Gel Paste compositions containing the Silicone organic elastomer*

The gel compositions of the present invention can be used to prepare gel paste compositions by;

I) shearing the silicone organic elastomer gel, as described above,
II) combining the sheared silicone organic elastomer gel with additional quantities of ii) the carrier fluid, as described above, and optionally
E) a personal or health care active active
to form a gel paste composition.

The silicone organic elastomer gel compositions of the present invention may be considered as discrete crosslinked silicone organic elastomers dispersed in carrier fluids. The silicone organic elastomer gel compositions are also effective rheological thickeners for many organic and silicone fluids. As such they can be used to prepare useful gel blend compositions, such as "paste" compositions.

To make such silicone organic elastomer pastes, the aforementioned silicone organic elastomer gels of known initial elastomer content are sheared to obtain small particle size may optionally be further diluted to a final elastomer content. "Shearing", as used herein refers to any shear mixing process, such as obtained from homogenizing, sonalating, or any other mixing processes known in the art as shear mixing. The shear mixing of the silicone organic elastomer gel composition results in a composition having reduced particle size. The subsequent composition having reduced particle size is then further combined with additional quantities of ii) the carrier fluid. Typically, the amount of carrier fluid added to the gel to form the gel paste is sufficient to provide a gel paste composition containing 30 wt % of the silicone organic elastomer, alternatively 20 wt %, or alternatively 10 wt%. The carrier fluid may be any carrier fluid as described above. In one embodiment, the carrier fluid is an aliphatic hydrocarbon, such as those described above. In another embodiment, the carrier fluid is an organopolysiloxane having a viscosity at 25°C in the range of 1 to 1,000 $mm^2$/sec.

The technique for combining the ii) carrier fluid with the silicone organic elastomer composition, and typically involves simple stirring or mixing. The resulting compositions may be considered as a paste, having a viscosity at least 50 Pa·s, alternatively at least 100 Pa·s, or alternatively at least 200 Pa·s, as measured on a Brookfield DVII+ viscometer with Helipath attachment using spindle T-D (20.4 mm crossbar) at 2.5 rpm.

## Examples

These examples are intended to illustrate the invention to one of ordinary skill in the art and should not be interpreted as limiting the scope of the invention set forth in the claims. All measurements and experiments were conducted at 23°C, unless indicated otherwise.

## Materials

*Organohydrogensiloxane 1* = a dimethyl, methylhydrogen polysiloxane having an average formula of $(CH_3)_3SiO[(CH_3)_2SiO]_x[(CH_3)HSiO]_ySi(CH_3)_3$, where x and y are of a value such that the organohydrogensiloxane has a viscosity of 116 $mn^2$/s (cSt) at 23°C and contains 0.084 wt. % H as Si-H.

*Organohydrogensiloxane 2* = a dimethyl, methylhydrogen polysiloxane having an average formula of $(CH_3)_3SiO[(CH_3)_2SiO]_x[(CH_3)HSiO]_ySi(CH_3)_3$, where x and y are of a value such that the organohydrogensiloxane has a viscosity of 67 (cSt) at 23°C and contains 0.15 wt. % H as Si-H.

*Organohydrogensiloxane 3* = a dimethyl, methylhydrogen polysiloxane having an average formula of $(CH_3)_3SiO[(CH_3)_2SiO]_x[(CH_3)HSiO]_ySi(CH_3)_3$, where x and y are of a value such that the organohydrogensiloxane has a viscosity of 147 $mm^2$/s (cSt) at 25°C and contains 0.0814 wt. % H as Si-H.

*Organohydrogensiloxane 4* = a SiH terminal Dimethyl polysiloxane having an average formula of $H(CH_3)_2SiO[(CH_3)_2SiO]_xSi(CH_3)_2H$. where x is of a value such that the organohydrogensiloxane contains 0.067 wt. % H as Si-H.

*Organohydrogensiloxane 5* = a SiH terminal dimethyl polysiloxane having an average formula of $H(CH_3)_2SiO[(CH_3)_2SiO]_xSi(CH_3)_2H$, where x is of a value such that the organohydrogensiloxane contains 0.137 wt. % H as Si-H.

*Organovinylsiloxane* = a vinyl terminal dimethyl polysiloxane having the formula $(ViMe_2SiO_{0.5})_2(Me_2SiO)_x$ where x is of a value such that the organovinylsiloxane has a viscosity of 5 $mm^2$/s (cSt) at 23°C.

*Polyalkyloxylenes* having either one or two terminal allyl groups and varying amounts of propylene or ethylene oxide units were used in these example as summarized in the table below. The di-allyl functional polyalkylene oxides were synthesized from the corresponding OH terminal polyalkylene oxides by reaction with NaH to form the alkoxide, which was then reacted with allyl chloride to form the allyl terminal polyether. Alternately, the di-allyl functional polyalkylene oxides may be purchased commercially.

| Polyalkyloxylene # | Average structure | MW (g/mol) | Vinyl concentration (mmol/g) |
|---|---|---|---|
| *Polyalkyloxylene 1* | $CH_2=CHCH_2O(CH_2CH(CH_3)O)_{7.05}CH_2CH=CH_2$ | 505.9 | 3.95 |
| *Polyalkyloxylene 2* | $CH_2=CHCH_2O(CH_2CH(CH_3)O)_{17.3}CH_2CH=CH_2$ | 1086.5 | 1.84 |
| *Polyalkyloxylene 3* | $CH_2=CHCH_2O(CH_2CH(CH_3)O)_{34.6}CH_2CH=CH_2$ | 2073.9 | 0.96 |
| *Polyalkyloxylene 4* | $CH_2=CHCH_2O(CH_2CH(CH_3)O)_{17}OBu$ | 1134.7 | 1.97 |
| *Polyalkyloxyene 5* | $CH_2=CHCH_2O(CH_2CH_2O)_{17}OCH_3$ | 837.0 | 5.06 |

(continued)

| Polyalkyloxylene # | Average structure | MW (g/mol) | Vinyl concentration (mmol/g) |
|---|---|---|---|
| *Polyalkyloxylene 6* | $CH_2=CHCH_2O(CH_2CH(CH_3)O)_{11.2}CH_2CH=CH_2$ | 749.2 | 2.67 |

Example 1

*Preparation of a Gel containing Polyalkyloxylene Crosslinked Silicone organic elastomer*

First, 42.40 g (35.5 mmol Si-H) of *Organohydrogensiloxane 1*, 9.83 g of *Polyalkyloxylene 1*, and 194 g of isododecane were weighed into a 16 oz wide mouth jar containing a teflon coated stir bar. Then, the jar was sealed and heated to 70°C using either a water bath or oven. The jar was removed from heat, and then while stirring, 0.6 g of SYLOFF 4000 catalyst (0.52 wt. % platinum) was added to provide 12 ppm platinum. The jar was capped, placed in a 70°C water bath, and stirring continued until the reaction mixture gelled. The mixture was held at 70°C in either a water bath or oven for an additional 3 hours to provide a gel containing 21.1% silicone-organic elastomer.

Gels containing polyalkyloxylene crosslinked silicone organic elastomers having varying amounts of propylene oxide units (17.3 or 34.6) were synthesized using the procedure as described above. The formulations used to prepare these gel compositions are summarized in Table 1.

**Table 1**

| Example # | 1A | 1B | 1C |
|---|---|---|---|
| Gel Formulations | | | |
| *Organohydrogensiloxane 1* | 42.40 g | 34.94 g | 26.86 g |
| *Polyalkyloxylene 1* | 9.83 g | | |
| *Polyalkyloxylene 2* | | 17.26 g | |
| *Polyalkyloxylene 3* | | | 25.34 g |
| Isododecane | 194.4 g | 194.4 g | 194.4 g |
| Syloff 4000 - Pt catalyst | 0.6 g | 0.6 g | 0.6 g |

Example 2 (comparative)

*Preparation of a Gel containing Hexadiene Crosslinked Silicone organic elastomer*

First, 50.32 g (42.2 mmol Si-H) of *Organohydrogensiloxane 1*, 1.88 g (46.3 meq unsaturation) of 1,5-hexadiene, and 194 g of isododecane were weighed into a 16 oz wide mouth jar containing a stir bar. The 8.5 % excess of olefin (Si-H:Vi = 0.92) was intended to minimize the amount of residual Si-H. The jar was sealed and then heated to 70°C in an oven. The jar was removed from heat, and then while stirring, 0.6 g of SYLOFF 4000 catalyst (0.52 wt. % platinum) was added. This brought the mixture up to 12 ppm platinum. The jar was capped and then placed in a 70°C water bath where stirring continued until gelation. The mixture was held at 70°C in an oven for 3 hours to form the gel containing 21.1% elastomer.

Example 3

*Preparation of Gel Pastes from Elastomer Gels*

The elastomer gels, as made in Example 1 and Example 2, were made into gel pastes using high shear mixing. The shear steps included the addition of isododecane, *organovinylsiloxane*, and octyl methoxycinnamate (OMC) to product the pastes shown in Tables 2-5. The materials in Table 2 were sheared in a Waring Commercial Laboratory Blender. In shear step 1, the gel was sheared for 20 seconds at setting 1, then 20 seconds at setting 3, then 20 seconds at setting 5. Isododecane and an organovinylsiloxane were added followed by shearing for 30 seconds at each of the following settings: 1, 2, 3, 3. Between each setting, the material was scraped from the sides of the mixer cup using a spatula. Before the third shear step, additional isododecane and octyl methoxycinnamate were added according to Table 2 followed by 20 second shearing at each of the following settings: 1. 1, 1. Between each setting, the material was scraped from the sides of the mixer cup. The materials in Tables 3-5 were sheared using a Hauschild Speed Mixer model DAC 150 FVZ purchased from FlackTek Inc. In the first shear step, the gel was sheared for 25 seconds on the maximum setting (approximately 3500rpm). Isododecane and an organovinylsiloxane were added follower by shearing for 25 seconds on the maximum setting. Additional isododecane and octyl methoxycinnamate were added and the material was sheared for 25 seconds on the maximum setting.

**Table 2: Hexadiene-Crosslinked Silicone organic elastomer Paste Processing with Addition of OMC**

| Elastomer Blend Paste Processing | | 0%v OMC | 6%v OMC | 12%v OMC | 18%v OMC |
|---|---|---|---|---|---|
| shear step 1 | Example 2 gel (g) | 59.3 | 59.3 | 59.3 | 59.3 |
| shear step 2 | isododecane(g) | 4.1 | 4.1 | 4.1 | 4.1 |
| | organovinyisiloxane (g) | 0.5 | 0.5 | 0.5 | 0.5 |
| shear step 3 | octyl methoxycinnamate (g) | 0.0 | 6.2 | 12.3 | 18.5 |
| | isododecane (g) | 15.0 | 10.4 | 5.8 | 1.3 |

**Table 3: Diallyl Polyether-Crosslinked (7DP) Silicone organic elastomer Paste Processing with Addition of OMC**

| Elastomer Blend Paste Processing | | 0%v OMC | 6%v OMC | 12%v OMC | 18%v OMC |
|---|---|---|---|---|---|
| shear step 1 | Example 1 A gel (g) | 59.7 | 59.7 | 59.7 | 59.7 |
| shear step 2 | isododecane (g) | 4.1 | 4.1 | 4.1 | 4.2 |
| | organovinylsiloxane (g) | 0.5 | 0.5 | 0.5 | 0.5 |
| shear step 3 | octyl methoxycinnamate (g) | 0.0 | 6.3 | 12.4 | 18.5 |
| | isododecane (g) | 15.0 | 10.4 | 5.8 | 1.3 |

**Table 4: Diallyl Polyether-Crosslinked (17DP) Silicone organic elastomer Paste Processing with Addition of OMC**

| Elastomer Blend Paste Processing | | 0%v OMC | 6%v OMC | 12%v OMC | 18%v OMC |
|---|---|---|---|---|---|
| shear step 1 | Example 1 B gel (g) | 59.8 | 59.8 | 59.8 | 59.8 |
| shear step 2 | isododecane (g) | 4.1 | 4.1 | 4.1 | 4.1 |
| | organovinylsiloxane (g) | 0.5 | 0.5 | 0.5 | 0.5 |
| shear step 3 | octyl methoxycinnamate (g) | 0.0 | 6.2 | 12.4 | 18.5 |
| | isododecane(g) | 15.0 | 10.5 | 5.8 | 1.3 |

**Table 5: Diallyl Polyether-Crosslinked (35DP) Silicone organic elastomer Paste Processing with Addition of OMC**

| Elastomer er Blend Paste Processing | | 0%v OMC | 6%v OMC | 12%v OMC | 18%v OMC |
|---|---|---|---|---|---|
| shear step 1 | Example 1C gel (g) | 59.7 | 59.7 | 59.8 | 59.7 |
| shear step 2 | isododecane (g) | 4.1 | 4.1 | 4.1 | 4.1 |
| | organovinylsiloxane (g) | 0.5 | 0.5 | 0.5 | 0.5 |

(continued)

| Elastomer er Blend Paste Processing | | 0%v OMC | 6%v OMC | 12%v OMC | 18%v OMC |
|---|---|---|---|---|---|
| shear step 3 | octyl methoxycinnamate (g) | 0.0 | 6.2 | 12.3 | 18.5 |
| | isododecane (g) | 15.0 | 10.4 | 5.9 | 1.3 |

Example 4

*Compatibility of Gels containing a Polyalkyloxylene Crosslinked Silicone organic elastomer, with octyl methoxycinnamate*

Organic compatibility of the silicone organic elastomer gels may be improved by increasing the degree of polymerization (DP) of the polypropylene glycol (PPG) crosslinker, as demonstrated in this example. When using the same organohydrogensiloxane, increasing the number of propylene oxide units in the polyalkaxylene increases the organic content of the elastomeric component and organic compatibility, as demonstrated in this example using octyl methoxycinnamate, an organic sunscreen known to have limited solubility with silicone organic elastomer gels. As summarized in Table 6, the compatibility of the silicone organic elastomer with octyl methoxycinnamate increases as the propylene oxide content increases (as denoted by DP or degree of polymerization, i.e. number of propylene oxide units), as based on clarity.

Viscosity changes also provided an indication of compatibility. As shown in Table 7, viscosity decreases with higher octyl methoxycinnamate levels in the hexadiene-crosslinked elastomer pastes while viscosity was maintained or increased in the polypropylene glycol crosslinked elastomer pastes. The viscosities were determined on a Brookfield DV-II+ Rheometer with the Helipath attachment and T-D spindle (t-bar geometry) at 2.5 RPM. Viscosities were determined one day after the samples were synthesized. The samples were vacuum de-aired and allowed to sit undisturbed for a minimum of four hours prior to testing. The data were acquired during two cycles of a down and up path through the sample. The reported viscosity was an average of the first upward and second downward pass of the t-bar through the sample.

**Table 6**

**Compatibility (Clarity) of Elastomer Blends as a Function of the Crosslinker and Volume Percent OMC**

| | Volume % OMC | | | |
|---|---|---|---|---|
| *Crosslinker* | *0* | *6* | *12* | *18* |
| Hexadiene | A | B | D | E |
| 7 DP PPG | A | A | A | C |
| 17 DP PPG | A | A | A | B |
| 34 DP PPG | A | A | A | A |

*A = transparent, C= translucent, E = hazy*

**Table 7**

| Viscosity of Elastomer Blends as a Function of the Crosslinker and Volume Percent OMC | | | | |
|---|---|---|---|---|
| *Crosslinker* | Paste Viscosity (*10^3cP) by Helipath Viscometer: 2.5rpm, Spindle T-D | | | |
| | 0% OMC | 6% OMC | 12% OMC | 18% OMC |
| Hexadiene | 367 | 339 | 242 | 70 |
| 7 DP PPG | 464 | 493 | 521 | 512 |
| 17 DP PPG | 546 | 663 | 762 | >800 |
| 34 DP PPG | 430 | 550 | 607 | 692 |

Example 5

*Compatibility of Gel Pastes containing a Polyalkyloxylene Crosslinked Silicone organic elastomer with various personal ingredients*

Table 8 summarizes compatibility data for a silicone-organic elastomer gel paste based on a polypropylene glycol crosslinker formulated with a variety of personal care ingredients at a 15 wt % addition level vs. a silicone organic elastomer gel paste based on hexadiene crosslinker. Compatibility was based on assessing both appearance (clarity and homogeneity) and thickening behaviors. Table 9 shows similar compatibility tests but using 25 wt % actives. As summarized in these tables, compatibility improved by incorporating polypropylene glycol into the elastomer.

**Table 8**
**Compatibility of Elastomer Gel Pastes in Isododecane Swelling Agent with 15 wt% Organic Actives**

| Cross-linker | Crosslinker as % of Elastomer | Octyl Methoxy-cinnamate | Ethanol | C12-15 Alkyl Benzoate | Caprylic/ Capric Triglyceride | Sun-flower Oil | Octyl Salicylate | Squalane | PPG-15 Stearyl Ether |
|---|---|---|---|---|---|---|---|---|---|
| Hexadiene | ~3.6 | C | B | B | B | D | B | B | D |
| PPG | 20.5 | A | A | A | A | B | A | A | A |

A = highly compatible, B = slight incompatibility. C = marginal Compatibility. D = incompatible. Rating based on observed clarity and thickening behaviors.

**Table 9**

| Compatibility of Elastomer Gel Pastes in Isododecane Swelling Agent with 25% Organic Actives | | | | | | | | | |
|---|---|---|---|---|---|---|---|---|---|
| Cross-linker | Crosslinker as % of Elastomer | Octyl Methoxy-cinnamate | Ethanol | C12-15 Alkyl Benzoate | Caprylicl Capric Triglyceride | Sun-flower Oil | Octyl Salicylate | Squalane | PPG-15 Stearyl Ether |
| Hexadiene | ~3.6 | D | D | C | D | D | B | D | D |
| PPG | 20.5 | B | B | A | A | D | A | D | D |

A = highly compatible. B = slight incompatibility, C = marginal compatibility, D = incompatible. Rating based on observed clarity and thickening behaviors

Example 6

*Sensory Behavior of Gels containing a Polyalkyloxylene Crosslinked Silicone Organic elastomer*

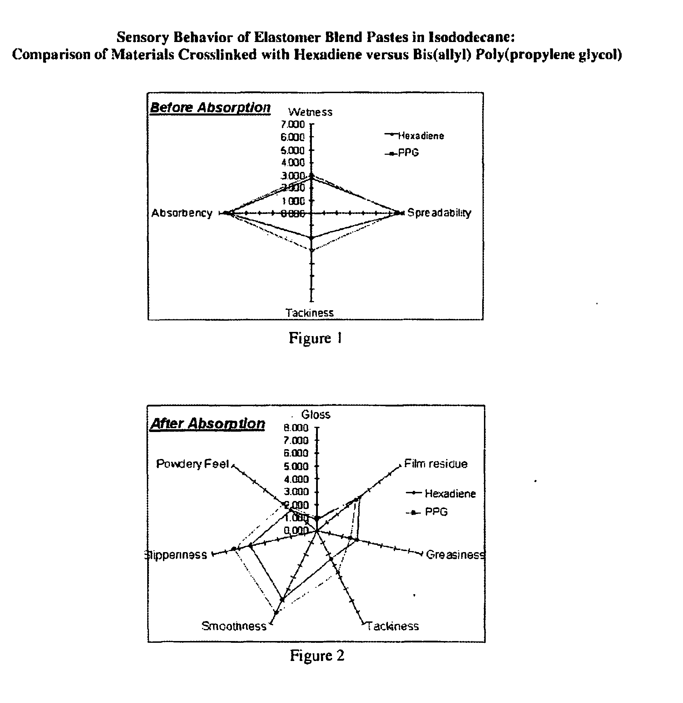

The sensory aesthetics of neat elastomer gel pastes were evaluated using a trained expert sensory panel. The resulting sensory data is shown in Figures 1 and 2, which compares the sensory aesthetics of a polyalkyloxylene crosslinked silicone organic elastomer paste vs. a hexadiene crosslinked silicone organic elastomer paste. The results show the sensory profiles are similar.

Example 7

*Preparation of a Gel containing Polyalkyloxylene Crosslinked Silicone organic elastomer having pendent polyoxyethylene groups*

Organohydrogensiloxane 2, monoallyl polyether groups [either poly(propylene oxide) or poly(ethylene oxide), polyalkyoxylene 4 or 5] and isododecane were charged into the reaction vessel and heated to 70°C. While stirring, 0.7g of a solution of 4000 catalyst (Karstedt's catalyst Platinum (0) complexed with 1,3-divinyl-1,1,3,3-tetramethyldisiloxane) was added. The reaction mixture was stirred at 70°C for 15 minutes. A polyalkyloxylene crosslinker (#2) was then added to the reaction mixture. The final reaction mixture was held at 70°C for three hours during which time a gel was formed. Actual gel formulations are shown in Tables 10 and 11. The gels were made into pastes using shear and additional solvent as shown in Table 12.

Table 10

| Formulation for bisallyl polyether crosslinked gel with poly(propylene oxide) pendent groups | | |
|---|---|---|
| Combine these three materials and heat to 70 °C in a capped glass jar | 25.9 g | Organohydrogensiloxane 2 |
| | 10.1 g | Polyalkyloxylene 4 |
| | 185.6 g | isododecane |
| While stirring, add platinum catalyst | 0.7 g | 4000 Catalyst (Pt) |
| 15min after catalyst addition, add these two materials while stirring | 20.2 g | Polyalkyloxylene 2 |
| | 13.2g | isododecane |
| Hold reaction mixture at 70 °C for 3 hr. | | |

Table 11

| Formulation for bisallyl polyether crosslinked gel with poly(ethylene oxide) pendent groups | | |
|---|---|---|
| Combine these three materials and heat to 70 °C in a capped glass jar | 29.1 g | Organohydrogensiloxane 2 |
| | 4.4 g | Polyalkyloxylene 5 |
| | 185.6 g | isododecane |
| While stirring, add platinum catalyst | 0.7 g | 4000 Catalyst (Pt) |
| 15min after catalyst addition, add these two materials while stirring | 22.6 g | Polyalkyloxylene 2 |
| | 13.2 g | isododecane |
| Hold reaction mixture at 70 °C for 3 hr. | | |

Table 12

| Formulation for silicone organic elastomer pastes processed from gels in Tables 10 and 11 | | |
|---|---|---|
| shear step 1 | 56.9 g | silicone/polyether elastomer gel |

(continued)

| Formulation for silicone organic elastomer pastes processed from gels in Tables 10 and 11 | | |
|---|---|---|
| shear step 2 | 26.2 g | isododecane |
| | 0.5 g | organovinylsiloxane |

The pastes were mixed with water (2 parts paste, 1 part water). The paste with poly(ethylene oxide) pendent groups formed a water-in-oil emulsion, while the paste with poly(propylene oxide) pendent groups did not form an emulsion.

### Example 8

*Modifying the Elastomer Network for Improved Compatibility with Organic Additives*

This example demonstrates that crosslink density and elastomer composition in the gel may be changed by replacing a portion of a siloxane having pendent SiH groups with a siloxane having terminal SiH groups. Modification to organic compatibility of the cosmetic pastes made from these gels may be achieved in this fashion.

First, 9.50 g (7.67 mmol Si-H) of *Organohydrogensiloxane 3*, 3.30 g (8.81 meq unsaturation) of *Polyalkyloxylene 6*, 67.20 g of isododecane and 60 microliters of platinum catalyst solution were weighed into an 8 oz wide mouth jar containing a teflon coated stir bar. The jar was sealed and placed into a 70°C water bath for 3 hours. Stirring was maintained until the mixture gelled. The result was a gel containing 16.0 wt. % of a silicone-organic elastomer in isododecane. The gel was then sheared to form a paste and diluted with additional isododecane to form a paste having 12.5 wt. % elastomer. Small amounts of a vinyl functional siloxane and triphenyl phosphine were added during the shear step to eliminate residual SiH and inhibit residual platinum. Similar elastomers were made in which a portion of the SiH pendent siloxane was replaced with SiH terminal siloxanes, which acts to modify the crosslink density as well as the wt. % poly(propylene oxide) in the copolymer. Compatibility of the samples was evaluated by mixing the diluted elastomer with squalane, sunflower oil, or octyl methoxycinnimate. The compatibility samples were evaluated visually for clarity and viscosity, where higher clarity and higher viscosity were considered indicative of improved compatibility. Compatibility samples exhibiting low viscosity were pourable liquids, whereas those judged as high viscosity did not flow readily when the samples were inverted. The results are shown in Table 13.

**Table 13**

| Example # | 8A | 8B | 8C |
|---|---|---|---|
| _Gel Formulations_ | | | |
| *Organohydrogensiloxane3* | 9.50 g | 7.36 g | 22.82 g |
| *Organohydrogensiloxane4* | | 2.23 g | |
| *Organohydrogensiloxane5* | | | 5.83 g |
| *Polyalkyloxylene 6* | 3.30 g | 3.21 g | 11.36 |
| Isododecane | 67.20 g | 67.20 g | 210.06 |
| Pt Catalyst Solution | 60 μL | 60 μL | 187 μL |
| Clarity*/Vicosity | | | |
| With 25% Squalane | 2 layers | C/Low | B/High |
| With 25% Sunflower Oil | 2 layers | D/Low | C/High |
| With 25% OMC | C/High | B/High | High |
| * A = transparent, C = translucent, E = hazy | | | |

### Example 9

*Compatibility of Gel Pastes with Octyl Methoxycinnamate where the gel contains Polyalkyloxylene Crosslinked Silicone organic elastomer in a silicone carrier fluid*

Examples 1-8 show the utility of using polyalkyloxylene crosslinked silicone organic elastomers in the organic carrier fluid isododecane. This example demonstrates that improved compatibility with an organic active can also be achieved when the polyalkyloxylene crosslinked silicone organic elastomer is made in a silicone carrier fluid as compared to a hexadiene crosslinked silicone elastomer in a silicone carrier fluid.

First, materials 1-3 in Table 14 and a Teflon coated stir bar were charged into a 16oz. wide mouth glass jar. The jar was sealed and the contents were heated to 70°C in a water bath. The jar was removed from the water bath and materials 4-6 in Table 14 were charged while stirring the contents of the jar. The jar was sealed and placed in a 70°C water bath with stirring. After 3 hours, the jar was removed from the water bath.

**Table 14**

| Material # | Material | Sample 9A | Sample 9B |
|---|---|---|---|
| 1 | Organohydrogensiloxane 1 | 27.67g | 41.43g |
| 2 | Polyalkyloxylene 2 | 15.31g | |
| 3 | Decamethylcyclopentasiloxane | 286.11g | 266.19g |
| 4 | Decamethylcyclopentasiloxane | | 19.93g |
| 5 | 1,5-hexadiene | | 1.55g |
| 6 | Syl-Off ® 4000 catalyst | 0.58g | 0.58g |

The gels were then sheared into pastes using a Hauschild DAC 150 speed mixer according to Table 15 and Table 16. Each gel was weighed into a 100g max mixer cup and sheared at 3450rpm for 2 runs of 30 seconds each (shear step 1). Additional decamethylcyclopentasiloxane and organovinylsiloxane were added and the gel was sheared again at 3450rpm for 2 runs of 30 seconds each (shear step 2). Octyl methoxycinnamate and additional decamethylcyclopentasiloxane were charged and the gel was sheared again at 3450rpm for 2 runs of 30 seconds each (shear step 3).

**Table 15**

| Shear step | Material | 0g OMC | 6g OMC | 12g OMC | 18g OMC |
|---|---|---|---|---|---|
| 1 | Gel 9A | 69.2g | 69.2g | 69.2g | 69.2g |
| 2 | decamethylcyclopentasiloxane | 5.2g | 5.2g | 5.3g | 5.3g |
| | Organovinylsiloxane | 0.4g | 0.4g | 0.4g | 0.4g |
| 3 | Octyl methoxycinnamate | | 6.1g | 12.1g | 18.2g |
| | decamethylcyclopentasiloxane | 18.2g | 12.6g | 7.1g | 1.4g |

**Table 16**

| Shear step | Material | 0g OMC | 6g OMC | 12g OMC | 18g OMC |
|---|---|---|---|---|---|
| 1 | Gel 9B | 69.2g | 69.2g | 69.2g | 69.2g |
| 2 | decamethylcyclopentasiloxane | 5.3g | 5.2g | 5.2g | 5.3g |
| | Organovinylsiloxane | 0.4g | 0.4g | 0.4g | 0.4g |
| 3 | Octyl methoxycinnamate | | 6.1g | 12.0g | 18.1g |
| | decamethylcyclopentasiloxane | 18.2g | 12.6g | 7.0g | 1.3g |

Viscosity (Brookfield-Helipath viscometer at 2.5rpm, spindle T-D) and clarity of the pastes were assessed as measures of compatibility such that loss of clarity and viscosity are considered indications of incompatibility. Table 17 shows that the inclusion of octyl methoxycinnamate results in decreases in viscosity for the hexadiene crosslinked elastomer, but results in an increase in viscosity for the polyalkyloxylene crosslinked elastomer. Similarly, clarity decreases with the addition of octyl methoxycinnamate for the hexadience crosslinked elastomer, but clarity is not negatively affected by the addition of octyl methoxycinnamate in the polyalkyloxylene crosslinked elastomer. Thus, even in a gel system where the carrier fluid is a silicone, improvements in compatibility with organic additives have been noted with the use of a polyalkyloxylene in the elastomer network.

**Table 17**

|  | 0g OMC | 6g OMC | 12g OMC | 18g OMC |
|---|---|---|---|---|
| Paste from 9A (polyalkyloxylene crosslinked) | 175,000cP | 215,000cP | 261,000cP | 322,000cP |
| Paste from 9B (hexadiene crosslinked) | 196,000cP | 197,000cP | 149,000cP | 84,000cP |

**Table 18**

|  | 0g OMC | 6g OMC | 12g OMC | 18g OMC |
|---|---|---|---|---|
| Paste from 9 A (polyalkyloxylene crosslinked) | B | B | B | A |
| Paste from 9B (hexadiene crosslinked) | A | A | D | F |

**Claims**

1. A composition comprising:

   i) 2 - 95 weight % of a silicone organic elastomer comprising a reaction product of;

   A) an organohydrogensiloxane comprising siloxy units of average formula

   $$(R^1_3SiO_{0.5})_v(R^2_2SiO)_x(R^2HSiO)_y$$

   wherein $R^1$ is hydrogen or $R^2$,
   $R^2$ is a monovalent hydrocarbyl,
   $v \geq 2$, $x \geq 0$, $y \geq 2$,
   B) a polyoxyalkylene comprising the average formula

   $$R^3O\text{-}[(C_2H_4O)_c(C_3H_6O)_d(C_4H_8O)_e]\text{-}R^3$$

   wherein
   $R^3$ is a monovalent unsaturated aliphatic hydrocarbon group containing 2 to 12 carbon atoms,
   c is from 0 to 50,
   d is from 0 to 100,
   e is from 0 to 100,
   with the proviso that the ratio of $(d + e)/(c + d + e)$ is greater than 0.5,
   C) a hydrosilylation catalyst, and

   ii) 5 - 98 weight % a carrier fluid,
   iii) optionally a personal care or healthcare active,

   wherein the composition is a gel.

2. The composition of claim 1 wherein the polyoxyalkylene is selected from

   $$H_2C=CHCH_2O[C_3H_6O]_dCH_2CH=CH_2$$

   $$H_2C=C(CH_3)CH_2O[C_3H_6O]_dCH_2C(CH_3)=CH_2$$

   $$HC{\equiv}CCH_2O[C_3H_6O]_dCH_2C{\equiv}CH, \text{ or}$$

   $$HC{\equiv}CC(CH_3)_2O[C_3H_6O]_dC(CH_3)_2C{\equiv}CH$$

   where d is from 1 to 100.

**EP 2 167 014 B1**

3. The composition of claims 1 or 2 wherein the carrier fluid is an organic liquid, preferably an aliphatic hydrocarbon or an organopolysiloxane having a viscosity at 25°C in the range of 1 to 1,000 $mm^2$/sec.

4. The composition of claim 1 wherein the molar ratio of the unsaturated aliphatic hydrocarbon groups in B) to the SiH units in A) is greater than 1.

5. The composition of claim 1 or 2 wherein A) the organohydrogensiloxane is selected from an organohydrogensiloxane having the average formula

$$(R^1_3SiO_{0.5})_v(R^2_2SiO)_x(R^2HSiO)_y(R^2SiO_{1.5})_z,$$

$$(R^1_3SiO_{0.5})_v(R^2_2SiO)_x(R^2HSiO)_y(SiO_2)_w,$$

$$(R^1_3SiO_{0.5})_v(R^2_2SiO)_x(R^2HSiO)_y(SiO_2)_w(R^2SiO_{1.5})_z$$

or any mixture thereof, where
$R^1$ is hydrogen or $R^2$,
$R^2$ is a monovalent hydrocarbyl, and $v \geq 2$, $w \geq 0$, $x \geq 0$, $y \geq 2$, and z is $\geq 0$, or
the organohydrogensiloxane is a dimethyl, methyl-hydrogen polysiloxane having the average formula;

$$(CH_3)_3SiO[(CH_3)_2SiO]_x[(CH_3)HSiO]_ySi(CH_3)_3$$

where $x \geq 0$, and $y \geq 2$, or
the organohydrogensiloxane is a mixture of organohydrogensiloxanes having the average formula $(CH_3)_3SiO[(CH_3)_2SiO]_x[(CH_3)HSiO]_ySi(CH_3)_3$ and $H(CH_3)_2SiO[(CH_3)_2SiO]_xSi(CH_3)_2H$
where $x \geq 0$, and $y \geq 2$.

6. The composition of claim 1 wherein the organohydrogensiloxane is a dimethyl, methyl-hydrogen polysiloxane having the average formula;

$$(CH_3)_3SiO[(CH_3)_2SiOM(CH_3)HSiO]_ySi(CH_3)_3$$

where $x \geq 0$, and $y \geq 2$,
the polyoxyalkylene is $H_2C=CHCH_2O[C_3H_6O]_dCH_2CH=CH_2$ where d is 1 to 100, and
the carrier fluid is an aliphatic hydrocarbon, preferably isododecane.

7. The composition of claim 1 wherein the silicone organic elastomer comprises a reaction product of;

A) an organohydrogensiloxane comprising siloxy units of average formula $(R^1_3SiO_{0.5})_v(R^2_2SiO)_x(R^2HSiO)_y$, wherein
$R^1$ is hydrogen or $R^2$,
$R^2$ is a monovalent hydrocarbyl,
$v \geq 2$, $x \geq 0$, $y \geq 2$,
B) a polyoxyalkylene comprising the average formula

$$R^3O-[(C_2H_{4O})_c(C_3H_6O)_d(C_4H_8O)_a]-R^3$$

wherein
$R^3$ is a monovalent unsaturated aliphatic hydrocarbon group containing 2 to 12 carbon atoms,
c is from 0 to 50,
d is from 0 to 100,
e is from 0 to 100,
with a proviso the ratio of (d + e)/(c + d + e) is greater than 0.5, C) a hydrosilylation catalyst, and
D) an organic compound having one terminal unsaturated aliphatic hydrocarbon group,

8. The composition of claim 7 wherein

D) is D') an alpha olefin or

D) is D") a polyoxyalkylene having the average formula

$$R^3O\text{-}[(C_2H_4O)_{c'}(C_3H_6O)_{d'}(C_4H_8O)_e]\text{-}R^4$$

where
$R^3$ is a monovalent unsaturated aliphatic hydrocarbon group containing 2 to 12 carbon atoms,
c', d', and e may vary from 0 to 100,
providing the sum of c', d', and e is > 0,
$R^4$ is hydrogen, an acyl group, or a monovalent hydrocarbon group containing 1 to 8 carbons.

9. A process for preparing a gel composition comprising reacting:

A) an organohydrogensiloxane comprising siloxy units of average formula

$$(R^1{}_3SiO_{0.5})_v(R^2{}_2SiO)_x(R^2HSiO)_y$$

wherein $R^1$ is hydrogen or $R^2$,
$R^2$ is a monovalent hydrocarbyl
$v \geq 2, x \geq 0, y \geq 2$,
B) a polyoxyalkylene comprising the average formula

$$R^3O\text{-}[(C_2H_4O)_c(C_3H_6O)_d(C_4H_8O)_e]\text{-}R^3$$

wherein
$R^3$ is a monovalent unsaturated aliphatic hydrocarbon group containing 2 to 12 carbon atoms,
c is from 0 to 50,
d is from 0 to 100,
e is from 0 to 100,
with the proviso that the ratio of (d + e)/(c + d + e) is greater than 0.5,
C) a hydrosilylation catalyst, and optionally
D') a hydrocarbon containing 6-30 carbons having one terminal unsaturated aliphatic hydrocarbon group,
D") a polyoxyalkylene having one terminal unsaturated aliphatic group, or mixtures of D') and D"),
in the presence of a carrier fluid, to form a gel.

10. A process for preparing a gel paste composition comprising;

I) shearing the gel composition of claim 1,
II) combining the sheared gel composition with additional quantities of ii) the carrier fluid to form a gel paste composition.

11. The process of claim 10 further comprising the addition of E) a personal care or healthcare active in step II).

12. The process of claim 11 wherein the personal care active is a sunscreen agent.

13. The process of claim 12 wherein the sunscreen agent is octyl methoxycinnamate.

14. The composition of claim 1 being a gel paste prepared according to the process of any of claims 10 -13.

15. The composition of claim 14 wherein the gel paste has a viscosity of at least 50 Pa·s.

**Patentansprüche**

1. Zusammensetzung enthaltend:

i) 2-95 Gew.-% eines siliconorganischen Elastomers, umfassend ein Reaktionsprodukt von

A) einem Organowasserstoffsiloxan mit Siloxyeinheiten der mittleren Formel

$$(R^1SiO_{0,5})_v(R^2_2SiO)_x(R^2HSiO)_y,$$

worin
$R^1$ Wasserstoff oder $R^2$ ist,
$R^2$ eine einbindige Kohlenwasserstoffgruppe ist, $v \geq 2$, $x \geq 0$, $y \geq 2$ ist,
B) einem Polyoxyalkylen mit der mittleren Formel

$$R^3O\text{-}[(C_2H_4O)_c(C_3H_6O)_d(C_4H_8O)_e]\text{-}R^3,$$

worin
$R^3$ eine einbindige ungesättigte aliphatische Kohlenwasserstoffgruppe mit 2 bis 12 Kohlenstoffatomen ist,
c von 0 bis 50 ist,
d von 0 bis 100 ist,
e von 0 bis 100 ist,
unter der Voraussetzung, dass das Verhältnis von (d + e)/(c + d + e) größer als 0,5 ist,
C) einem Hydrosilylierungskatalysator und

ii) 5 - 98 Gew.-% einer Trägerflüssigkeit,
iii) optional ein Körperpflege- oder Gesundheitsfürsorgemittel ist, wobei die Zusammensetzung ein Gel ist.

2. Zusammensetzung nach Anspruch 1, wobei das Polyoxyalkylen ausgewählt ist aus

$$H_2C=CHCH_2O[C_3H_6O]_dCH_2CH=CH_2$$

$$H_2C=C(CH_3)CH_2O[C_3H_6O]_dCH_2C(CH_3)=CH_2$$

$$HC\equiv CCH_2O[C_3H_6O]_dCH_2C\equiv CH \text{ oder}$$

$$HC\equiv CC(CH_3)_2O[C_3H_6O]_dC(CH_3)_2C\equiv CH, \text{ worin}$$

d von 1 bis 100 ist.

3. Zusammensetzung nach den Ansprüchen 1 oder 2, wobei die Trägerflüssigkeit eine organische Flüssigkeit ist, vorzugsweise ein aliphatischer Kohlenwasserstoff, oder ein Organopolysiloxan mit einer Viskosität bei 25°C im Bereich von 1 bis 1.000 mm$^2$/sec.

4. Zusammensetzung nach Anspruch 1, wobei das molare Verhältnis der ungesättigen aliphatischen Kohlenwasserstoffgruppen in B) zu den SiH-Einheiten in A) größer als 1 ist.

5. Zusammensetzung nach Anspruch 1 oder 2, worin A) das Organowasserstoffsiloxan ausgewählt ist aus einem Organowasserstoffsiloxan mit der mittleren Formel

$$(R^1_3SiO_{0,5})_v(R^2_2SiO)_x(R^2HSiO)_x(R^2SiO_{1,5})_z,$$

$$(R^1_3SiO_{0,5})_v(R^2_2SiO)_x(R^2HSiO)_y(SiO_2)_w,$$

$$(R^1_3SiO_{0,5})_v(R^2_2SiO)_x(R^2HSiO)_y(SiO_2)_w(R^2SiO_{1,5})_z$$

oder einer Mischung davon, worin
$R^1$ Wasserstoff oder $R^2$ ist,
$R^2$ eine einbindige Kohlenwasserstoffgruppe ist und $v \geq 2$, $w \geq 0$, $x \geq 0$, $y \geq 2$ und $z \geq 0$ ist oder
das Organowasserstoffsiloxan ein Dimethylmethyl-Wasserstoffpolysiloxan mit der mittleren Formel

$$(CH_3)_3SiO[(CH_3)_2SiO]_x[(CH_3)HSiO]_ySi(CH_3)_3 \text{ ist,}$$

worin $x \geq 0$ und $y \geq 2$ ist oder
das Organowasserstoffsiloxan eine Mischung aus Organowasserstoffsiloxanen mit der mittleren Formel

$$(CH_3)_3SiO[(CH_3)_2SiO]_x[(CH_3)HSiO]_ySi(CH_3)_3 \text{ und}$$

$$H(CH_3)_2SiO[(CH_3)_2SiO]_xSi(CH_3)_2H \text{ ist,}$$

worin $x \geq 0$ und $y \geq 2$ ist.

6.  Zusammensetzung nach Anspruch 1, worin das Organowasserstoffsiloxan ein Dimethylmethyl-Wasserstoffpolysiloxan mit der mittleren Formel

$$(CH_3)_3SiO[(CH_3)_2SiO]_x[(CH_3)HSiO]_ySi(CH_3)_3 \text{ ist,}$$

worin $x \geq 0$ und $y \geq 2$ ist,
das Polyoxyalkylen $H_2C=CHCH_2O[C_3H_6O]_dCH_2CH=CH_2$ ist, worin d gleich 1 bis 100 ist, und
die Trägerflüssigkeit ein aliphatischer Kohlenwasserstoff, vorzugsweise Isododecan ist.

7.  Zusammensetzung nach Anspruch 1, wobei das siliconorganische Elastomer ein Reaktionsprodukt aus

A) einem Organowasserstoffsiloxan mit Siloxyeinheiten der mittleren Formel

$$(R^1_3SiO_{0,5})_v(R^2_2SiO)_x(R^2HSiO)_y,$$

worin
$R^1$ Wasserstoff oder $R^2$ ist,
$R^2$ eine einbindige Kohlenwasserstoffgruppe ist, $v \geq 2$, $x \geq 0$, $y \geq 2$ ist,
B) einem Polyoxyalkylen mit der mittleren Formel

$$R^3O\text{-}[(C_2H_4O)_c(C_3H_6O)_d(C_4H_8O)_e]\text{-}R^3, \text{ worin}$$

$R^3$ eine einbindige ungesättigte aliphatische Kohlenwasserstoffgruppe mit 2 bis 12 Kohlenstoffatomen ist,
c von 0 bis 50 ist,
d von 0 bis 100 ist,
e von 0 bis 100 ist,
unter der Voraussetzung, dass das Verhältnis von $(d + e)/(c + d + e)$ größer als 0,5 ist,
C) einem Hydrosilylierungskatalysator und
D) einer organischen Verbindung mit einer endständigen ungesättigten aliphatischen Kohlenwasserstoffgruppe

enthält.

8.  Zusammensetzung nach Anspruch 7, worin

D) D') ein $\alpha$-Olefin ist oder
D) D") ein Polyoxyalkylen mit der mittleren Formel $R^3O\text{-}[(C_2H_4O)_{c'}(C_3H_6O)_{d'}(C_4H_8O)_e]\text{-}R^4$ ist, worin $R^3$ eine einbindige ungesättigte aliphatische Kohlenwasserstoffgruppe mit 2 bis 12 Kohlenstoffatomen ist, c', d' und e von 0 bis 100 variieren können, unter der Voraussetzung, dass die Summe von c', d' und e > 0 ist, $R^4$ Wasserstoff, eine Acylgruppe oder eine einbindige Kohlenwasserstoffgruppe mit 1 bis 8 Kohlenstoffatomen ist.

9.  Verfahren zur Herstellung einer Gelzusammensetzung, umfassend Umsetzen von:

A) einem Organowasserstoffsiloxan mit Siloxyeinheiten der mittleren Formel

$$(R^1_3SiO_{0,5})_v(R^2_2SiO)_x(R^2HSiO)_y,$$

worin
$R^1$ Wasserstoff oder $R^2$ ist,
$R^2$ eine einbindige Kohlenwasserstoffgruppe ist, $v \geq 2$, $x \geq 0$, $y \geq 2$ ist,
B) einem Polyoxyalkylen mit der mittleren Formel

$$R^3O\text{-}[(C_2H_4O)_c(C_3H_6O)_d(C_4H_8O)_e]\text{-}R^3, \text{ worin}$$

$R^3$ eine einbindige ungesättigte aliphatische Kohlenwasserstoffgruppe mit 2 bis 12 Kohlenstoffatomen ist,
c von 0 bis 50 ist,
d von 0 bis 100 ist,
e von 0 bis 100 ist,
unter der Voraussetzung, dass das Verhältnis von (d + e)/(c + d + e) größer als 0,5 ist,
C) einem Hydrosilylierungskatalysator und optional D') einem Kohlenwasserstoff mit 6-30 Kohlenstoffatomen mit einer endständigen ungesättigten aliphatischen Kohlenwasserstoffgruppe,
D") einem Polyoxyalkylen mit einer endständigen ungesättigten aliphatischen Gruppe oder einer Mischung von D') und D"),

in Gegenwart einer Trägerflüssigkeit, um ein Gel zu bilden.

**10.** Verfahren zur Herstellung einer Gelpastenzusammensetzung, umfassend

I) Scheren der Gelzusammensetzung nach Anspruch 1,
II) Kombinieren der gescherten Gelzusammensetzung mit zusätzlichen Mengen von
ii) der Trägerflüssigkeit, um eine Gelpastenzusammensetzung zu bilden.

**11.** Verfahren nach Anspruch 10, das zusätzlich die Zugabe von E) einem Körperpflege- oder Gesundheitsfürsorgemittel in Schritt II) umfasst.

**12.** Verfahren nach Anspruch 11, wobei das körperpflegeaktive Mittel ein Sonnenschutzmittel ist.

**13.** Verfahren nach Anspruch 12, wobei das Sonnenschutzmittel Octylmethoxycinnamat ist.

**14.** Zusammensetzung nach Anspruch 1, die eine Gelpaste ist, die gemäß dem Verfahren nach einem der Ansprüche 10 -13 hergestellt ist.

**15.** Zusammensetzung nach Anspruch 14, wobei die Gelpaste eine Viskosität von mindestens 50 Pa·s aufweist.

**Revendications**

**1.** Composition comprenant :

i) 2-95 % en poids d'un élastomère organique de silicone comprenant un produit de la réaction de :

A) un organohydrogénosiloxane comprenant des unités siloxy de formule moyenne

$$(R^1{}_3SiO_{0,5})_v(R^2{}_2SiO)_x(R^2HSiO)_y$$

où $R^1$ est l'hydrogène ou $R^2$,
$R^2$ est un hydrocarbyle monovalent, $v \geq 2$, $x \geq 0$, $y \geq 2$,
B) un polyoxyalkylène comprenant la formule moyenne

$$R^3O\text{-}[(C_2H_4O)_c(C_3H_6O)_d(C_4H_8O)_e]\text{-}R^3$$

où
$R^3$ est un groupe hydrocarboné aliphatique insaturé monovalent contenant 2 à 12 atomes de carbone,
c est de 0 à 50,
d est de 0 à 100,
e est de 0 à 100,
avec la condition que le rapport de (d + e)/(c +d + e) soit supérieur à 0,5,
C) un catalyseur d'hydrosilylation, et

ii) 5-98 % en poids d'un fluide vecteur,

iii) éventuellement une substance active pour les soins personnels ou les soins de santé,

où la composition est un gel.

2. Composition selon la revendication 1 où le polyoxyalkylène est choisi parmi

$$H_2C=CHCH_2O[C_3H_6O]_dCH_2CH=CH_2$$

$$H_2C=C(CH_3)CH_2O[C_3H_6O]_dCH_2C(CH_3)=CH_2$$

$$HC\square CCH_2O[C_3H_6O]_dCH_2C\square CH, \text{ ou}$$

$$HC\square CC(CH_3)_2O[C_3H_6O]_dC(CH_3)_2C\square CH$$

où d est de 1 à 100.

3. Composition selon la revendication 1 ou 2 où le fluide vecteur est un liquide organique, de préférence un hydrocarbure aliphatique ou un organopolysiloxane ayant une viscosité à 25°C dans la plage de 1 à 1 000 mm$^2$/s.

4. Composition selon la revendication 1 où le rapport molaire des groupes hydrocarbonés aliphatiques insaturés dans B) aux unités SiH dans A) est supérieur à 1.

5. Composition selon la revendication 1 ou 2 où A) l'organohydrogénosiloxane est choisi parmi un organohydrogénosiloxane ayant la formule moyenne

$$(R^1{}_3SiO_{0,5})_v(R^2{}_2SiO)_x(R^2HSiO)_y(R^2SiO_{1,5})_z,$$

$$(R^1{}_3SiO_{0,5})_v(R^2{}_2SiO)_x(R^2HSiO)_y(SiO_2)_w,$$

$$(R^1{}_3SiO_{0,5})_v(R^2{}_2SiO)_x(R^2HSiO)_y(SiO_2)_w(R^2SiO_{1,5})_z$$

ou tout mélange de ceux-ci, où
$R^1$ est l'hydrogène ou $R^2$,
$R^2$ est un hydrocarbyle monovalent, et $v \geq 2$, $w \geq 0$, $x \geq 0$, $y \geq 2$, et $z$ est $\geq 0$, ou
l'organohydrogénosiloxane est un diméthyl, méthyl-hydrogénopolysiloxane ayant la formule moyenne :

$$(CH_3)_3SiO[(CH_3)_2SiO]_x[(CH_3)HSiO]_ySi(CH_3)_3$$

où $x \geq 0$ et $y \geq 2$, ou
l'organohydrogénosiloxane est un mélange d'organohydrogénosiloxanes ayant la formule moyenne $(CH_3)_3SiO[(CH_3)_2SiO]_x[(CH_3)HSiO]_ySi(CH_3)_3$ et $H(CH_3)_2SiO[(CH_3)_2SiO]_xSi(CH_3)_2H$
où $x \geq 0$, et $y \geq 2$.

6. Composition selon la revendication 1 où l'organohydrogénosiloxane est un diméthyl, méthyl-hydrogénopolysiloxane ayant la formule moyenne :

$$(CH_3)_3SiO[(CH_3)_2SiO]_x[(CH_3)HSiO]_ySi(CH_3)_3$$

où $x \geq 0$, et $y \geq 2$,
le polyoxyalkylène est $H_2C=CHCH_2O[C_3H_6O]_dCH_2CH=CH_2$ où d est 1 à 100, et
le fluide vecteur est un hydrocarbure aliphatique, de préférence l'isododécane.

7. Composition selon la revendication 1 où l'élastomère organique de silicone comprend un produit de la réaction de :

A) un organohydrogénosiloxane comprenant des unités siloxy de formule moyenne

$$(R^1{}_3SiO_{0,5})_v(R^2{}_2SiO)_x(R^2HSiO)_y$$

où $R^1$ est l'hydrogène ou $R^2$,
$R^2$ est un hydrocarbyle monovalent, $v \geq 2$, $x \geq 0$, $y \geq 2$,
B) un polyoxyalkylène comprenant la formule moyenne

$$R^3O\text{-}[(C_2H_4O)_c(C_3H_6O)_d(C_4H_8O)_e]\text{-}R^3$$

où
$R^3$ est un groupe hydrocarboné aliphatique insaturé monovalent contenant 2 à 12 atomes de carbone,
c est de 0 à 50,
d est de 0 à 100,
e est de 0 à 100,
avec la condition que le rapport de (d + e)/(c +d + e) soit supérieur à 0,5,
C) un catalyseur d'hydrosilylation, et
D) un composé organique ayant un groupe hydrocarboné aliphatique insaturé en position terminale.

8. Composition selon la revendication 7 où

D) est D') une alpha oléfine ou
D) est D") un polyoxyalkylène ayant la formule moyenne

$$R^3O\text{-}[(C_2H_4O)_{c'}(C_3H_6O)_{d'}(_4H_6O)_e]\text{-}R^4 \text{ où}$$

$R^3$ est un groupe hydrocarboné aliphatique insaturé monovalent contenant 2 à 12 atomes de carbone,
c', d' et e peuvent varier de 0 à 100,
à condition que la somme de c', d'et e soit > 0, $R^4$ est l'hydrogène, un groupe acyle ou un groupe hydrocarboné monovalent contenant 1 à 8 carbones.

9. Procédé pour préparer une composition de gel comprenant la réaction :

A) d'un organohydrogénosiloxane comprenant des unités siloxy de formule moyenne

$$(R^1{}_3SiO_{0,5})_v(R^2{}_2SiO)_x(R^2HSiO)_y$$

où $R^1$ est l'hydrogène ou $R^2$,
$R^2$ est un hydrocarbyle monovalent,
$v \geq 2$, $x \geq 0$, $y \geq 2$,
B) d'un polyoxyalkylène comprenant la formule moyenne

$$R^3O\text{-}[(C_2H_4O)_c(C_3H_6O)_d(C_4H_8O)_e]\text{-}R^3$$

où

$R^3$ est un groupe hydrocarboné aliphatique insaturé monovalent contenant 2 à 12 atomes de carbone, c est de 0 à 50,
d est de 0 à 100,
e est de 0 à 100,
avec la condition que le rapport de (d + e)/(c +d + e) soit supérieur à 0,5,
C) d'un catalyseur d'hydrosilylation, et et éventuellement
D') d'un hydrocarbure contenant 6-30 carbones ayant un groupe hydrocarboné aliphatique insaturé en position terminale,
D") d'un polyoxyalkylène ayant un groupe aliphatique insaturé en position terminale, ou des mélanges de D') et D"),

en présence d'un fluide vecteur, pour former un gel.

10. Procédé pour former une composition de pâte de gel comprenant :

I) le cisaillement de la composition de gel selon la revendication 1,

II) la combinaison de la composition de gel cisaillée avec des quantités supplémentaires de
ii) le fluide vecteur pour former une composition de pâte de gel.

11. Procédé selon la revendication 10 comprenant en outre l'addition de

E) une substance active pour les soins personnels ou les soins de santé dans l'étape II).

12. Procédé selon la revendication 11 où la substance active pour les soins personnels est un agent d'écran solaire.

13. Procédé selon la revendication 12 où l'agent d'écran solaire est le méthoxycinnamate d'octyle.

14. Composition selon la revendication 1 qui est une pâte de gel préparée selon le procédé selon l'une quelconque des revendications 10-13.

15. Composition selon la revendication 14 où la pâte de gel a une viscosité d'au moins 50 Pa.s.

EP 2 167 014 B1

**Sensory Behavior of Elastomer Blend Pastes in Isododecane:**
**Comparison of Materials Crosslinked with Hexadiene versus Bis(allyl) Poly(propylene glycol)**

Figure 1

Figure 2

29

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5880210 A
- US 5760116 A
- US 5811487 A
- US 6200581 B
- US 5236986 A
- US 6331604 B
- US 6262170 B
- US 6531540 B
- US 6365670 B
- US 2001041771 A1, KONDO HIDETOSHI
- US 6168782 B1, LIN ZUCHEN
- US 3419593 A, Willing
- US 5175325 A, Brown
- US 3989668 A, Lee
- US 5036117 A, Chang
- US 3159601 A, Ashby
- US 3220972 A, Lamoreaux
- US 3296291 A, Chalk
- US 3516946 A, Modic
- US 3814730 A, Karstedt
- US 3928629 A, Chandra
- US 01722901 A
- US 6987157 B

**Non-patent literature cited in the description**

- United States Department of Health & Human Services Food and Drug Administration. 200-299300-499